# EUROPEAN PATENT APPLICATION

(11) **EP 4 682 539 A1**
(43) Date of publication of application: **21.01.2026**
(21) Application number: 24188718.1
(22) Date of filing: 15.07.2024
(51) Int. Cl.: G01N 33/58, G01N 33/68

(54) **METHOD FOR DETECTING MISFOLDED ALPHA-SYNUCLEIN PROTEIN IN BIOLOGICAL SAMPLES TO DETERMINE THE PRESENCE OF SYNUCLEINOPATHIES IN AN INDIVIDUAL**

(71) Applicant: Modag GmbH, 55234 Wendelsheim (DE)
(72) Inventor: Longen, Dr. Sebastian, 67061 Ludwigshafen am Rhein (DE); Troßbach, Dr. Svenja, 65199 Wiesbaden (DE); Weckbecker, Dr. Daniel, 81375 München (DE); Matthias, Dr. Torsten, 55237 Flonheim (DE); Schmidt, Dr. Felix, 81541 München (DE); Giese, Dr. Armin, 69198 Schriesheim (DE)
(74) Representative: Schaafhausen, Anne

(57) **Abstract**

The invention relates to a method for detecting misfolded alpha-synuclein protein in a biological sample to determine the presence of synucleinopathies in an individual. The method provided by the present invention can also be used for differential diagnosis of Lewy fold synucleinopathies from MSA synucleinopathies as well as for monitoring of the disease progression in individuals. Moreover, a kit for detecting misfolded αSyn protein in biological samples is provided.

## Description

The present invention relates to a method for detecting misfolded alpha-synuclein protein in a biological sample to determine the presence of synucleinopathies in an individual.

Aberrant proteostasis and protein misfolding may lead to numerous diseases. Especially, protein misfolding in the brain can severely damage brain cells and lead to neurodegenerative disorders (NDs). NDs are a broad category of disorders characterized by the progressive degeneration of neurons and other cells in the brain and/or spinal cord. This degeneration typically leads to a decline in cognitive function, movement, and/or other neurological functions. Presently, many NDs are not curable and difficult to diagnose. In some cases, a final diagnosis can only be made *post mortem* by neuropathological examination of the brain. All common NDs are characterized by the misfolding, aggregation, and deposition of specific proteins in the brain. Examples of NDs include Alzheimer's disease (AD), Creutzfeldt Jakob disease (CJD), or synucleinopathies such as Parkinson's disease (PD), Lewy body dementia (DLB), and multiple system atrophy (MSA). The misfolded protein, the cell type, and the brain region the aggregation occurs in as well as the clinical symptoms determine the respective disease. Even though the biological origin of NDs is still not fully understood, many share as a pathogenic mechanism that proteins change their native conformation and form pathological β-sheet-containing fibrils. These fibrils can subsequently serve as templates for their native counterparts and thus, promote further fibril formation, cell death, and spreading.

Synucleinopathies refer to a group of NDs characterized by the abnormal accumulation of a protein called alpha-synuclein (αSyn) within brain cells. αSyn is a well conserved, small acidic protein of 140 amino acids with a molecular weight of 14 kDa. Synucleinopathies are associated with the formation of abnormal αSyn protein fibrils inside nerve cells and other cell populations within the central nervous system (CNS). The most well-known synucleinopathy is PD, but other disorders fall under this category as well, including Parkinson's disease dementia (PDD), DLB, MSA, and pure autonomic failure (PAF). Based on structural differences as presented by the conformation of the αSyn fibrils, synucleinopathies can be divided into two subcategories, namely the Lewy fold strains (PD, PDD, and DLB) and the MSA strain. These conditions share some common features, such as motor and/or cognitive impairments as well as autonomic dysfunction, but they also have distinct clinical and pathological characteristics. Neuropathologically, synucleinopathies can be distinguished from other NDs by the disease-specific accumulation and deposition of aggregated and misfolded αSyn protein, whereas other NDs are characterized by the accumulation and deposition of other aggregated and misfolded proteins. For example, CJD is characterized by aggregated and misfolded prion protein (PrP^{Sc}) and AD by Abeta (Aβ) and tau protein.

In the context of NDs that are not primary synucleinopathies, such as clinical and neuropathological AD, it is well known that Lewy fold synuclein deposits can be found as co-pathologies. These co-pathologies might play an important role in disease progression and in the clinical phenotype.

Currently, diagnosing synucleinopathies is challenging due to the overlap in symptoms of different synucleinopathies and other NDs and the lack of an objective biological diagnostic test. Diagnosis of PD or DLB typically involves a combination of medical history review, physical examination, neurological assessment, and various diagnostic tests such as neuroimaging to assess if clinical criteria are met. Thus, diagnosing synucleinopathies requires a comprehensive evaluation by a neurologist or movement disorder specialist, incorporating multiple clinical and diagnostic factors to arrive at an accurate diagnosis. However, the diagnostic accuracy of a clinical diagnosis of PD, for example, varies between 26% and 88%. Thus, as no diagnostic test that detects the underlying Lewy fold pathology is approved for clinical use at present, there is a need in the art to provide such a test.

Biochemical tests for the detection of misfolded proteins have been known since 2001, when the first assay for the detection of pathological prion protein fibrils in CJD was described. In principle, these assays have in common that they comprise at least two steps: a) inducing the elongation of the (initially patient-derived) pathological protein seeds with native protein monomers as substrate into fibrils and b) fragmenting the fibrils to generate more seeds. These alternating two steps are repeated multiple times to increase the concentration of pathological protein fibrils in order to obtain detectable concentrations. Due to this principle, these assays have previously been known as PMCA (protein misfolding cyclic amplification) and RT-QuIC (real-time quaking-induced conversion), both recently combined under the term SAA (seed amplification assay or seeding aggregation assay). The assay has been adapted for synucleinopathies, namely the αSyn SAA, with which the presence of pathological αSyn seeds in biological samples can be detected. In the assay, a patient-derived biological sample such as cerebrospinal fluid (CSF) that may contain misfolded αSyn seeds is added to a reaction buffer containing recombinantly generated monomeric αSyn as a substrate. If the patient sample contains misfolded αSyn seeds, they can induce the aggregation of the monomeric protein into fibrils, effectively "seeding" further aggregation. αSyn fibrils comprise a β-sheet structure to which a fluorophore such as thioflavin T (ThT) can bind with high affinity and, after binding, exhibits a specific fluorescence at 480 nm. As the concentration of the pathological fibrils in the original biological sample is too low to detect, the fluorescence signal needs to be artificially increased by the described process. Via cyclic shaking and rest phases the fibrillar structure is broken up, thereby multiplying the number of potential aggregation nuclei. Over time, the fibrils multiply, which can be monitored by an increase in fluorescence signals in patients with synucleinopathies. As individuals who do not suffer from synucleinopathies have no initial seeds in their samples, no increase in fluorescence signal is occurring.

Due to the intrinsic capability of αSyn to form fibrils, one big challenge is to avoid the occurrence of spontaneous self-aggregation of αSyn during the assay progression, leading to false positive signals. This might be one reason why most of the assays described in the art have a cut-off after 30 - 40 hours. Moreover, self-aggregation makes it more difficult to clearly differentiate between positive and negative samples specifically. In the prior art, usually a final concentration of monomeric αSyn between 0.1-1 mg/ml is used in the reaction mixture of the assay.

In general, an αSyn SAA reaction is characterized by an increase in fluorescence due to an increase in αSyn fibrils. The curve characteristics are an initial lag phase followed by the onset of exponential growth of αSyn fibrils, namely the elongation phase. The reaction is represented by several assay kinetic parameters such as the maximum fluorescence intensity (Fₘₐₓ) observed, area under curve (AUC), time to threshold (TTT), or T₅₀ (time to reach 50% of the maximum fluorescence). By utilizing endpoint titration of the patient sample, an SD₅₀ (the dilution at which 50% of the replicates are still positive) can be calculated. There have also been attempts in the art to investigate if those kinetic parameters correlate with clinical parameters such as the Montreal Cognitive Assessment (MoCA), the Hoehn and Yahr (H&Y) scale, and MDS-UPDRS III (Movement Disorders Society - Unified Parkinson's Disease Rating Scale, Part 3). However, the outcomes were inconclusive.

Thus, there is a need to provide a highly sensitive, specific, and reliable assay to determine synucleinopathies in individuals. Further, there is a need to provide an assay to support the differential diagnosis of Lewy fold type synucleinopathies (PD, PDD, and DLB) and MSA-type synucleinopathies. In addition, there is a need to provide an assay for the discrimination of patients having a synucleinopathy from patients having another ND, to detect an αSyn co-pathology in other NDs, to draw conclusions about seed load, and to allow for tracking disease progression and/or treatment efficiency in patients having a synucleinopathy.

Therefore, the present invention is based on the object to provide a method for the detection of misfolded αSyn protein in a biological sample to determine the presence of synucleinopathies in an individual. The method provided by the present invention can also be used for differential diagnosis of Lewy fold synucleinopathies from MSA synucleinopathies as well as for monitoring of the disease progression in individuals. Moreover, a kit for detecting misfolded αSyn protein in biological samples is provided.

This object is solved by a method having the features according to claim 1, and a kit having the features of claim 17.

Preferred embodiments of the invention are defined in the respective dependent claims.

According to the invention, a method for detecting misfolded αSyn protein in a biological sample to determine the presence of synucleinopathies in an individual is provided, the method comprises the steps of:
- contacting a biological sample of the individual with a reaction buffer which comprises monomeric αSyn and a fluorophore, to form a reaction mixture;
- incubating the reaction mixture with intermittent shaking/resting cycles;
- subjecting the reaction mixture to excitation;
- determining the fluorescence intensity of the reaction mixture.

According to a preferred embodiment, the biological sample is a body fluid sample or a body tissue sample.

According to another preferred embodiment, the body fluid sample is selected from cerebrospinal fluid (CSF), blood, serum, plasma, saliva, urine, feces, lymph, preferably CSF.

According to another preferred embodiment, the body tissue sample is selected from mucosa, nasal swap, skin biopsy, *post mortem* brain homogenate.

Further, according to a preferred embodiment, the method comprises using replicates, preferably doublets, triplets, or quadruplets of the biological sample.

According to another preferred embodiment, the fluorescence intensity is determined every 45 to 75 minutes, preferably every 55 to 65 minutes, more preferably every 60 minutes.

According to yet another preferred embodiment, the reaction mixture comprises 100 µl total volume. Preferably, the amount of the biological sample in the reaction mixture is 2 - 25%, more preferably 5 - 20%, and most preferably 15%.

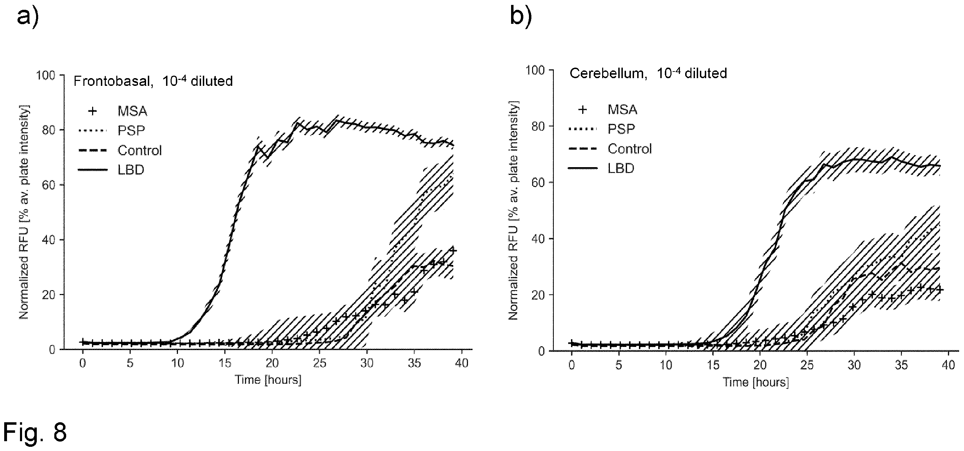

Surprisingly, the inventors were able to show that an increase in the number of false positive replicates was detected when using less than 15% CSF sample. This may be due to the fact that CSF has an inhibiting effect on the reaction. In addition, the inventors were able to show that using brain homogenate extricated *post mortem* from patients, only 2% of a diluted tissue sample was sufficient for the assay to work (see Figure 8).

According to still another preferred embodiment, the reaction buffer further comprises beads.

According to still another preferred embodiment, the beads comprise zirconia, silica, glass, quartz, and/or combinations thereof, wherein glass beads are preferred.

Further, according to a preferred embodiment, the beads have a mean diameter in the range of 0.01 mm to 1 mm, preferably of 0.1 to 0.9 mm, more preferably of 0.2 mm to 0.85 mm, and most preferably about 0.8 mm.

According to yet another preferred embodiment, the reaction mixture comprises 1 to 8 beads per reaction, preferably 2 to 7 beads per reaction, and more preferably 6 beads per reaction.

According to yet another preferred embodiment, the reaction buffer comprises a pH in the range of pH 6.6 - pH 9, preferably pH 7.0 - pH 8.5, more preferably pH 8.0.

Surprisingly, it was found in the present invention that significantly more spontaneous self-aggregation of αSyn occurred at a pH of < 6.6.

According to yet another preferred embodiment, the monomeric αSyn in the reaction buffer is a recombinant wildtype (WT) human αSyn, a variant thereof, or a fragment thereof.

The sequence of the WT human αSyn is as set forth in SEQ ID No: 1.

According to a preferred embodiment, the monomeric αSyn is not a mutant comprising one or more mutations. In particular, it had been shown that the method according to the invention could not be carried out with an αSyn mutant that was described in the prior art to provide faster results with a high sensitivity and specificity compared to WT αSyn. The inventors could show that when using the αSyn mutant of the prior art in the inventive method, a strong reduction of Fₘₐₓ and a decreased sensitivity was observed.

According to a preferred embodiment, the monomeric αSyn comprises six additional histidine amino acids on either its C-terminus or its N-terminus, i.e., a polyHis purification tag (polyHis tag, Hiss tag). According to a still more preferred embodiment, the monomeric αSyn comprises an N-terminal polyHis tag. In another preferred embodiment, the monomeric αSyn also comprises a linker. The sequence of the monomeric αSyn comprising a N-terminal polyHis tag and a linker is as set forth in SEQ ID No: 2.

In the meaning of the present invention, a linker is a short amino acid segment within a protein, connecting two domains.

According to a preferred embodiment, the concentration of the monomeric αSyn in the reaction buffer is in the range of 0.01 mg/ml - 0.095 mg/ml, preferably 0.03 mg/ml - 0.09 mg/ml, more preferably 0.04 mg/ml - 0.08 mg/ml, and most preferably 0.07 mg/ml.

Surprisingly, the inventors were able to show that a concentration of monomeric αSyn in the reaction buffer ≤ 0.05 mg/ml led to a strong reduction of the fluorescence signal. Further, at a concentration of ≥ 0.09 mg/ml monomeric αSyn in the reaction buffer, an increase in spontaneous self-aggregation of αSyn was observed. Thus, the present invention shows that monomeric αSyn concentrations < 0.1 mg/ml are stabilizing the assay's specificity overall by significantly reducing spontaneous self-aggregation without losing assay sensitivity.

According to another preferred embodiment, the fluorophore comprises one or more of: thioflavin T (ThT), thioflavin S (ThS), Congo Red, Thiazole Orange, SYBR Green, Crystal Violet, 1-anilino-8-naphthalene sulfonate (ANS), and the like. A still more preferred fluorophore is ThT.

According to another preferred embodiment, the concentration of the fluorophore in the reaction buffer is in the range of 2 µM to 25 µM, preferably 2.5 µM to 20 µM, more preferably 5 µM to 15 µM, still more preferably 7 to 11 µM, and most preferably 8 µM. Surprisingly, the inventors were able to show that the fluorescence signal strength decreases with increasing fluorophore concentration > 25 µM. It is assumed that this could be due to a kind of self-quenching whereby the fluorophores could sterically hinder each other.

According to another preferred embodiment, an increase in the fluorescence intensity indicates the presence of misfolded αSyn protein in the biological sample.

According to yet another preferred embodiment, a replicate is considered positive if the Fₘₐₓ reaches 5 - 30% of the average Fₘₐₓ of a positive control, preferably 10 - 25%, more preferably 15 - 20%, even more preferably 15%, and most preferably 20%.

According to a preferred embodiment, the reaction buffer comprises NaCl in the range of 10 mM - 500 mM, preferably 20 mM - 400 mM, more preferably 50 mM - 200 mM, and most preferably 150 mM.

According to still another preferred embodiment, the reaction buffer comprises sodium phosphate (NaPᵢ) in a concentration in the range of 10 mM to 250 mM. The minimum concentration of NaPᵢ is 10 mM, 20 mM, or 30 mM, and the maximum concentration of NaPᵢ is 150 mM, 200 mM, or 250 mM. Preferably, the concentration of NaPᵢ is 35 mM.

Surprisingly, the inventors showed that NaPᵢ achieved better results than HEPES (4-(2-hydroxyethyl)-1-piperazineethanesulfonic acid) buffer. The lag phase was significantly longer using HEPES.

According to a preferred embodiment, the reaction buffer comprises sodium dodecyl sulfate (SDS) in a concentration of 0.0008% to 0.0030%, preferably 0.0010% to 0.0026%, more preferably 0.0013%.

In the present invention, it could surprisingly be found that the lag phase, maximal fluorescence (Fₘₐₓ), as well as an increased occurrence of false positive and/or false negative results depend on the concentration of SDS in the reaction buffer, even though the critical micelle concentration of SDS in aqueous solutions is 0.2% and, thus, above the concentrations used in the present invention. In more detail, according to the present invention, increasing the concentration of SDS > 0.002% promotes the conversion of native monomeric protein to pathological species leading to a shorter lag phase and a higher Fₘₐₓ but also to a decreased specificity. Decreasing the SDS concentration < 0.0008% in the reaction mixture impairs the SAA reaction leading to a longer lag phase, reduced Fₘₐₓ and decreased sensitivity.

Since both the lag phase and Fₘₐₓ are strongly influenced by the concentration of SDS, a suitable concentration of SDS must be chosen. Surprisingly, concentrations around 0.0013% SDS have been shown to be the most effective in the inventive method.

According to another preferred embodiment, the reaction mixture is incubated for 30 - 144 hours, preferably 60 - 144 hours, more preferably 72 - 144 hours, and most preferably 144 hours. Also preferred is an incubation of 72 hours, and still more preferred of 96 hours.

Surprisingly, the inventors of the present invention were able to show that negative control samples did stay negative for 144 hours incubation using the inventive method. Over the course of 144 hours incubation, the inventors surprisingly showed that no self-aggregation of monomeric αSyn was observed using the inventive method.

According to still another preferred embodiment, the method is carried out at a temperature of 30 to 46°C, preferably 36 to 44°C, more preferably 42°C.

In the meaning of the present invention, the assay was performed by intermittent shaking and rest cycles. Thus, the samples were shaken at 200 to 800 rpm, preferably 300 to 600 rpm, more preferably 400 rpm. Shaking was performed for 1 to 5 minutes, preferably 1 to 3 minutes, and more preferably 1 minute. The rest phase was performed for 1 to 5 minutes, preferably 1 to 3 minutes, more preferably 1 min. According to a preferred embodiment, shaking and resting are substantially equal in length. Further, shaking was performed orbitally or double-orbitally, preferably double-orbitally.

Further, according to a preferred embodiment, the synucleinopathies are Lewy fold specific synucleinopathies.

According to still another preferred embodiment, the Lewy fold specific synucleinopathies are selected from Parkinson's disease (PD), Parkinson's Disease Dementia (PDD), and Dementia with Lewy Bodies (DLB).

According to another preferred embodiment, before the step of contacting a biological sample of the individuum with a reaction buffer, the method further comprises:
- diluting the biological sample in a negative control sample.

For the purpose of the invention, the negative control sample can be, for example, a biological CSF sample that has been tested negative for αSyn SAA, or another fluid that has been tested negative for αSyn SAA which is suitable for carrying out the SAA assay according to the invention.

Further, according to a preferred embodiment, the dilution series comprises undiluted biological sample and dilutions of 1:2 and/or 1:3 and/or 1:4 and/or 1:8 and/or 1:10 and/or 1:30 and/or 1:100.

Further, according to a preferred embodiment, the method comprises after the step of determining the fluorescence intensity of the reaction mixture:
- counting the number of positive signals obtained from the dilution series of the biological sample and scoring these numbers to obtain a Lewy fold pathology (LFP) score;
- correlating the scores to clinical parameters.

These above-mentioned method steps are suitable to provide a quantitative method.

Further, according to a preferred embodiment, the clinical parameters comprise UPDRS III (Unified Parkinson's Disease Rating Scale), MoCA (Montreal Cognitive Assessment) and/or Hoehn and Yahr (symptom progression in PD).

According to another aspect of the invention, a kit for detecting misfolded αSyn in a biological sample is provided, wherein the kit comprises an aqueous buffered solution comprising monomeric αSyn, a variant thereof or a fragment thereof.

According to another preferred embodiment, the kit comprises means to perform the method according to the above-mentioned embodiments.

According to still another preferred embodiment, the kit further comprises an aqueous buffered solution comprising sodium phosphate, an SDS solution, a NaCl solution and water, filter, beads, a fluorophore, a positive control, a negative control, one or more well plate(s) and an adhesive foil for covering of the one or more plate(s).

According to another preferred embodiment, the fluorophore is selected from the fluorophores as mentioned above.

In the meaning of the present invention, the term "fibril" refers to filamentous protein assemblies with a β-sheet structure, formed by the ordered aggregation of monomeric protein.

In the meaning of the present invention, the term "seeds" refers to misfolded αSyn protein that is originally present in the patient-derived sample before the elongation of added monomeric αSyn occurs in the αSyn SAA. The term "seed" further refers to misfolded αSyn proteins that are generated by the shaking/resting cycles of the αSyn SAA assay which therefore also include converted αSyn substrate.

In the meaning of the present invention, the term "Lewy fold pathology (LFP) score" refers to a score that serves as an estimate of seed load in Lewy fold synucleinopathies.

In the meaning of the present invention, the term "determination" or "determining" refers to the process of measuring, calculating, or assessing a particular quantity, value, or characteristic. For example, the determination of a chemical concentration in a sample or the determination of the presence and/or severity of a medical condition through diagnostic tests.

In the meaning of the present invention, the term "diluting" refers to the process of decreasing the concentration of a solute in a solution usually by adding more solvent to the solution. In the present invention, diluting the biological sample may be done in form of a serial dilution. Thus, the dilution occurs stepwise either by using a constant dilution factor or by using a variable factor between dilutions.

In the meaning of the present invention, the term "positive control" refers to a sample that contains pathological seeds, thus, a positively tested patient sample or a sample containing artificial fibrils. In addition, the term "negative control" refers to a negatively tested patient sample containing no pathological seeds that therefore does not lead to a positive αSyn SAA reaction or to another fluid that has been tested negative for αSyn SAA which is suitable for carrying out the SAA assay according to the invention.

In the meaning of the present invention, a replicate is considered positive if the Fₘₐₓ reaches 5 - 30% of the average Fₘₐₓ of a positive control, preferably 10 - 25%, more preferably 15 - 20%, even more preferably 15%, and most preferably 20%.

In the meaning of the present invention, artificial fibrils are generated by *in vitro* aggregation of recombinant αSyn monomers in aqueous solution by continuous shaking at 37°C. The resulting fibrils can be sonicated to obtain a more homogenous solution in terms of fibril size. In αSyn SAAs according to the present invention, artificial fibrils may be used at a concentration of 75 nM - 75 fM, preferably 7.5 nM - 750 fM, and more preferably 7.5 pM.

In the meaning of the present invention, the term "about" refers to minor variations or deviations from the stated number as long as they do not significantly alter the number. It is generally understood that the term allows for a reasonable margin of error or tolerance.

In the meaning of the present invention, the term "variant" refers to a peptide or amino acid sequence that deviates from another amino acid sequence only in the substitution of one or several nucleotides having no impact on the amino acid the codon encodes. As is well known to a person skilled in the art, a three-nucleotide codon in a nucleic acid sequence specifies a single amino acid. A single amino acid may be encoded by different codons. Another possibility of providing a variant of αSyn is an exchange of amino acids by conservative amino acid substitution. A "conservative amino acid substitution" is one in which an amino acid residue is substituted by another amino acid residue having a side chain (R group) with similar chemical properties (e.g., similar charge or hydrophobicity). In general, a conservative amino acid substitution will not substantially change the functional properties of a protein. In cases where two or more amino acid sequences differ from each other by conservative substitutions, the percent of degree of similarity may be adjusted upwards to correct for the conservative nature of the substitution. Means for making this adjustment are well known to those of skill in the art. Examples of groups of amino acids that have side chains with similar chemical properties, and whose substitution of each other constitutes conservative substitutions, include:
1) aliphatic side chains: glycine, alanine, valine, leucine and isoleucine
2) aliphatic hydroxyl side chains: serine and threonine
3) amide-containing side chains: asparagine and glutamine
4) aromatic side chains: phenylalanine, tyrosine and tryptophane
5) basic side chains: lysine, arginine and histidine
6) acidic side chains: aspartate and glutamate, and
7) sulfur-containing side chains: cysteine and methionine.

Preferred conservative amino acid substitution groups are: valine-leucine-isoleucine, phenylalanine-tyrosine, lysine-arginine, alanine-valine, glutamate-aspartate, and asparagine-glutamine.

In the meaning of the present invention, the term "fragment" as used herein refers to a molecule other than a full length αSyn protein. Thus, a fragment of αSyn is intended to mean a protein of at least 10, 20, 30, 40, 50, 100, 120, 130, or 140 contiguous amino acids of the sequence of αSyn, or any integer there between. Preferred fragments are those which are capable of aggregation under the conditions of the inventive method.

It has also to be noted that aspects of the invention have been described with reference to different subject-matters. In particular, some aspects or embodiments have been described with reference to method type claims, whereas other aspects have been described with reference to product type claims (kit). However, a person skilled in the art will gather from the above and the following description that, unless otherwise notified, in addition to any combination between features belonging to one type of subject-matter, also any combination between features relating to different types of subject-matters is considered to be disclosed with this text. In particular, combinations between features relating to the method type claims and features relating to the product type claims are considered to be disclosed. In addition, features relating to one of the embodiments may be combined with other features of another embodiment, the drawings or the claims, where possible. The invention and embodiments thereof will be described below in further detail in connection with the drawing(s).
- Fig. 1: shows a schematic illustration of the αSyn SAA principle according to an embodiment of the invention.
- Fig. 2: shows a schematic illustration of the assay according to another embodiment of the invention.
- Fig. 3: shows a schematic illustration of the results of an exemplary αSyn SAA reaction according to another embodiment of the invention.
- Fig. 4: shows an exemplary table of the αSyn SAA sensitivity and specificity according to another embodiment of the invention.
- Fig. 5: shows a schematic illustration of a serial dilution for LFP score quantification according to another embodiment of the invention.
- Fig. 6a - c: show schematic illustrations of LFP score in association with disease severity scores displayed in box and scatter plots, respectively, according to another embodiment of the invention.
- Fig. 7: shows a schematic illustration of a longitudinal investigation of LFP scores in association with clinical measures of disease severity in PD patients.
- Fig. 8: shows a schematic illustration of the results of an exemplary αSyn SAA reaction according to another embodiment of the invention using brain homogenate as biological sample.

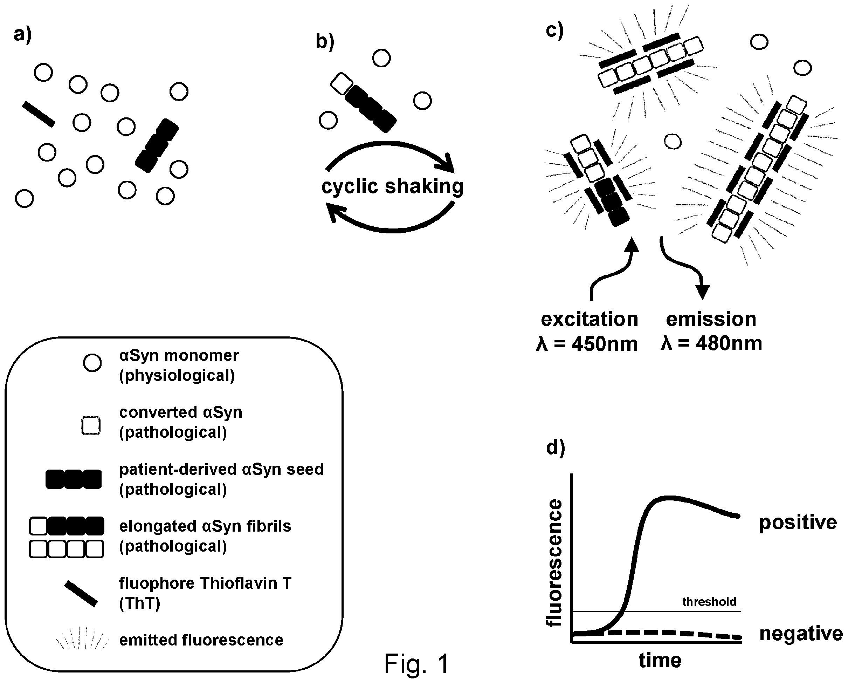

In Fig. 1, a schematic illustration of the assay principle is depicted. Pathological αSyn (fibrils, seeds) can serve as an aggregation template for physiological, monomeric αSyn (a). Thus, in the meaning of the present invention, pathological αSyn refers to misfolded αSyn protein, whereas physiological αSyn refers to native, monomeric αSyn protein. For the αSyn SAA according to the invention, samples of individuals which are to be examined for the presence of synucleinopathies are used in undiluted or undiluted and diluted form, in particular in the form of dilution series. A sample to be examined is contacted with a reaction buffer, also known as a master mix, to form a reaction mixture. The reaction buffer may contain the following components, for example:

| substrate | monomeric αSyn |
|---|---|
| substrate concentration | 0.07 mg/ml |
| NaCl | 150 mM |
| sodium phosphate | 35 mM |
| ThT | 8 µM |
| SDS | 0.0013% |
| pH | 8 |

The reaction mixture may be incubated for 72 - 144 h at 42°C. Intermittent shaking and rest cycles are carried out during the incubation (b). This typically involves shaking for 1 minute at 400 rpm followed by a rest phase of 1 minute. In the presence of misfolded αSyn (pathological) in the sample to be analyzed, αSyn monomers (physiological) react with the pathological form leading to an elongation of the fibril during the resting phase. Thereby, the physiological αSyn monomers provided as substrate are converted to β-sheet fibrils. In the shaking phase, these fibrils are broken up, increasing the number of pathological αSyn, which can then serve as new templates and support further aggregation in the next resting phase. Due to this cyclic shaking and resting phases, the seeds are multiplied over time (c). The fibril-specific fluorophore thioflavin T (ThT) specifically binds to the β-sheet fibrils and is used to monitor fibril growth over time (d). The fluorescence intensity can therefore be used to detect pathological αSyn in the sample to be analyzed.

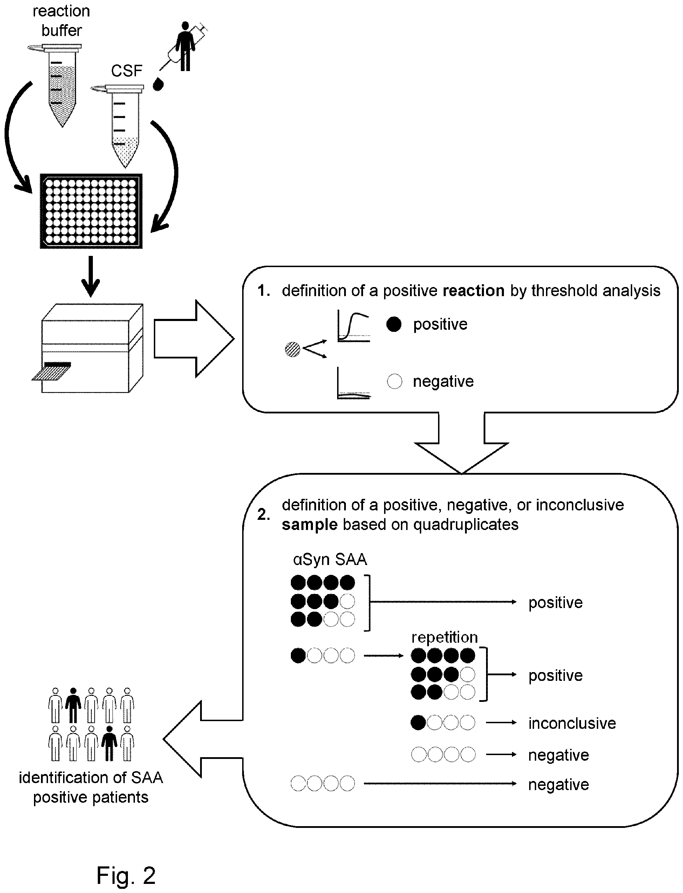

In Fig. 2, a schematic illustration of the assay procedure is shown. In this example, CSF samples were contacted with reaction buffer and measured in quadruplicates according to the inventive method. According to the invention, a replicate was considered positive or negative if a defined threshold of the maximal fluorescence intensity (Fₘₐₓ) was exceeded or not, in Figure 2, said threshold is 15% (box 1). As depicted above, each CSF sample was measured in quadruplicates, thus, four single replicates per sample were tested. A sample with 2, 3, or 4 positive replicates per quadruplicate was considered positive, samples with 4 negative replicates were considered negative. Samples with 1 positive replicate were considered inconclusive and the measurement was repeated. If the result of the repetition was again 1 positive replicate out of 4 replicates, the sample was declared inconclusive in conclusion (box 2). Thus, SAA positive patients can be identified on the basis of the inventive method.

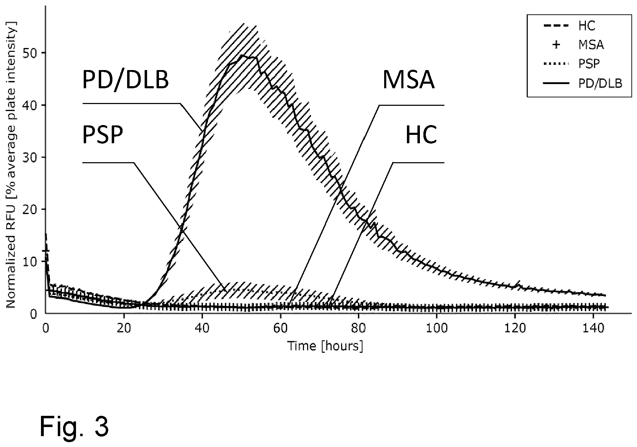

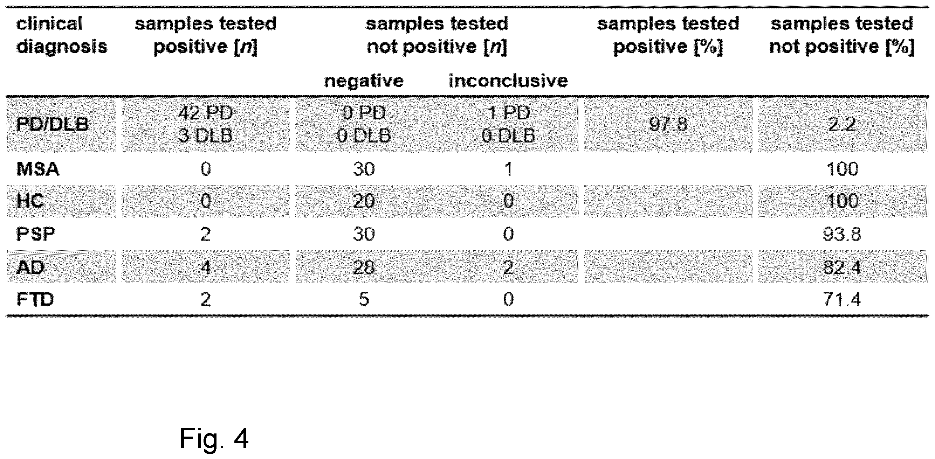

In Fig. 3, a schematic illustration of an exemplary αSyn SAA reaction is depicted, showing Lewy fold strain specificity over healthy control (HC), Progressive supranuclear palsy (PSP, a neurodegenerative disease other than a synucleinopathy) and MSA samples (PD/DLB, solid line, *n* = 46; MSA, + line, *n* = 31; PSP, dotted line, *n* = 32; HC, dashed line, *n* = 20). Each curve represents relative fluorescence unit (RFU) values of the group normalized to the maximum intensity of fluorescence of the respective experimental plate ± standard error of the mean (dashed pattern). Thus, the method according to the present invention is suitable for use in differential diagnostics of Lewy fold synucleinopathies from MSA-synucleinopathies. Fig. 3 and also Fig. 4 relate to CSF samples.

Fig. 4 shows an exemplary table of the αSyn SAA sensitivity and specificity. Depicted is the percentage of individuals tested positive, negative, or inconclusive of a cohort. The patients were initially clinically diagnosed with either PD, DLB, MSA, PSP, AD, or Frontotemporal Dementia (FTD). HC is the healthy control. As can be seen from the table, the method according to the present invention shows a 97.8% sensitivity for the Lewy fold-specific synucleinopathies PD and DLB, and a 100% specificity with respect to controls as well as samples of patients having MSA, allowing a differential diagnosis of PD/DLB and potential MSA. Also, some of the AD and FTD samples, being other NDs than synucleinopathies, showed positive signals for misfolded αSyn, indicating a co-pathology. Thus, the present invention is also able to determine a co-pathology in patients suffering from a ND other than a synucleinopathy.

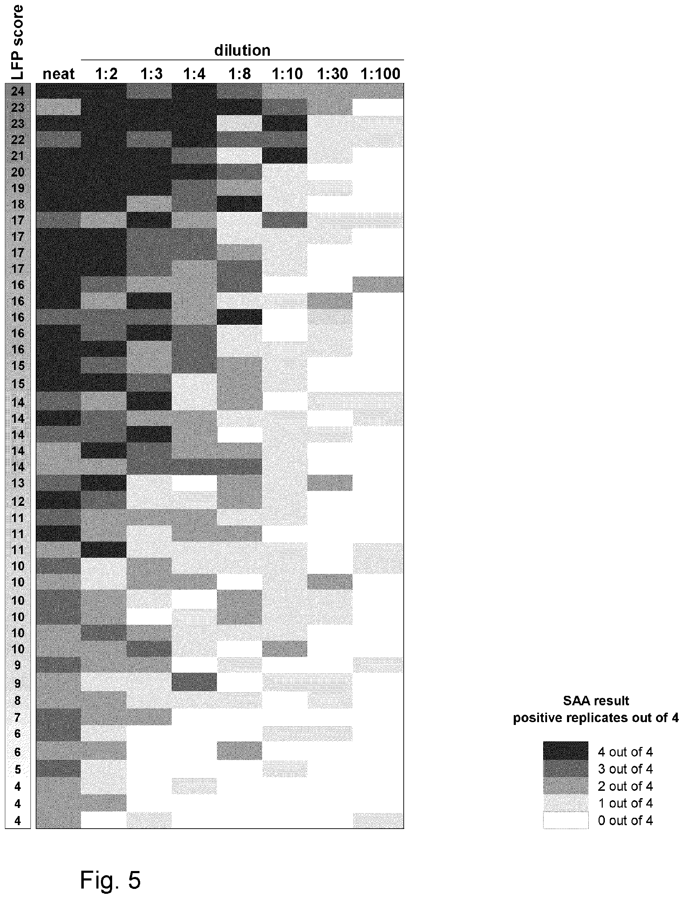

In Fig. 5, a schematic illustration of an LFP score obtained by a dilution series is depicted. In the prior art, a qualitatively comparable correlation of quantitative information with clinical progression data for synucleinopathies could not be demonstrated previously under the conditions of prior art αSyn SAAs and a subsequent dilution series in CSF as the biological sample. With the αSyn SAA according to the present invention, it was surprisingly found that a correlation of the seed load with clinical progression data is now possible for the first time using a dilution series and a subsequent LFP score analysis. The dilution series according to the invention may comprise undiluted samples (neat) and dilutions obtained from said samples in the ratios 1:2, 1:3, 1:4, 1:8, 1:10, 1:30, and 1:100. All samples tested in the dilution series shown in Fig. 5 were tested positive in the αSyn SAA according to the invention performed as depicted in Fig. 2 and have clinically diagnosed PD or DLB. In the dilution series, the total number of positive signals (LFP score) for each dilution as well as the undiluted samples was calculated and displayed as a heat map. Thus, black is 4 positive signals out of the 4 tested replicates, dark grey is 3 positive signals out of the 4 tested replicates, grey is 2 positive signals out of the 4 tested replicates, light grey is 1 positive signal out of the 4 tested replicates, and white is 0 positive signals out of the 4 tested replicates. According to the invention, the heat map may be correlated to the severity of the disease, meaning that samples with a high total number of positive signals (high LFP score) correspond to a high proportion of misfolded αSyn protein correlating to a high degree of severity of the disease or a later stage of the disease. A lower number of positive signals (lower LFP score) corresponds to a lower proportion of misfolded αSyn protein in the sample and, thus, to a lesser degree of severity of the disease or an earlier stage of the disease. Therefore, the dilution series according to the invention may be used to determine the degree of severity of the respective synucleinopathy.

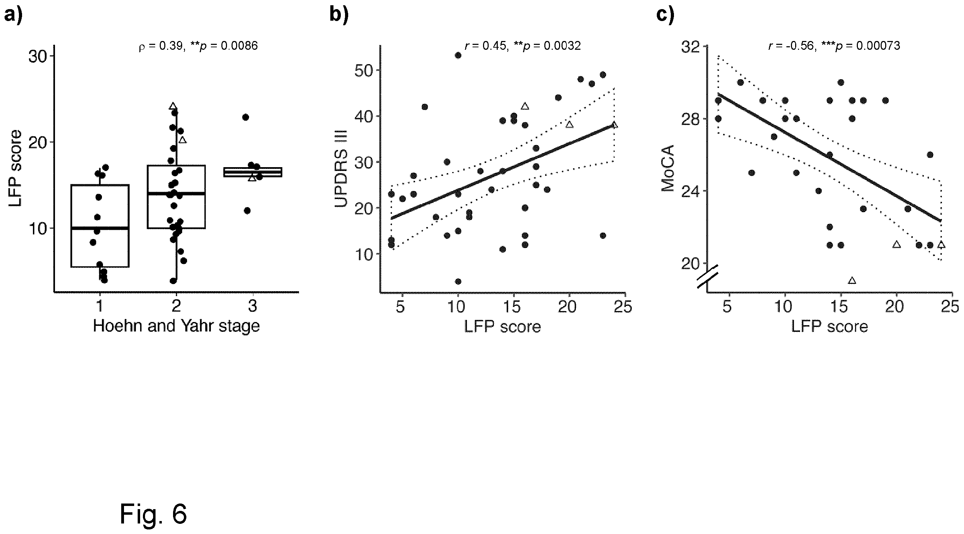

After assessing the LFP score of individual patients that showed positive signals in the initial (undiluted) αSyn SAA assay by serial dilution as described above, the LFP score was correlated with clinical data of PD/DLB cases (Fig. 6a -6c). In Fig. 6a - 6c, schematic illustrations of LFP score in association with disease severity scores are depicted. Fig 6a shows a schematic illustration of LFP score in association with the clinical scores of Hoehn and Yahr (symptom progression in PD) in a box plot. In Fig. 6b, the number of total positives was correlated to the clinical scores of UPDRS III (Unified Parkinson's Disease Rating Scale) in a scatter plot. In Fig. 6c, the number of total positives was correlated to the clinical scores of MoCA (Montreal Cognitive Assessment) displayed in a scatter plot.

It can clearly be seen that the Hoehn and Yahr Stage 1 to 3 at lumbar puncture (LP) can be correlated to the obtained results using the inventive αSyn SAA and the dilution series according to the invention. UPDRS is a rating scale for assessing the severity of PD. It is divided into 6 parts, wherein Part 3 is the assessment of gait disturbance by a physician. MoCA is a test to determine the severity of dementia. The Hoehn and Yahr scale is a classification of the severity of PD into 5 stages based on motor impairment. Hoehn and Yahr is included in the UPDRS and is listed there in Part V, among others.

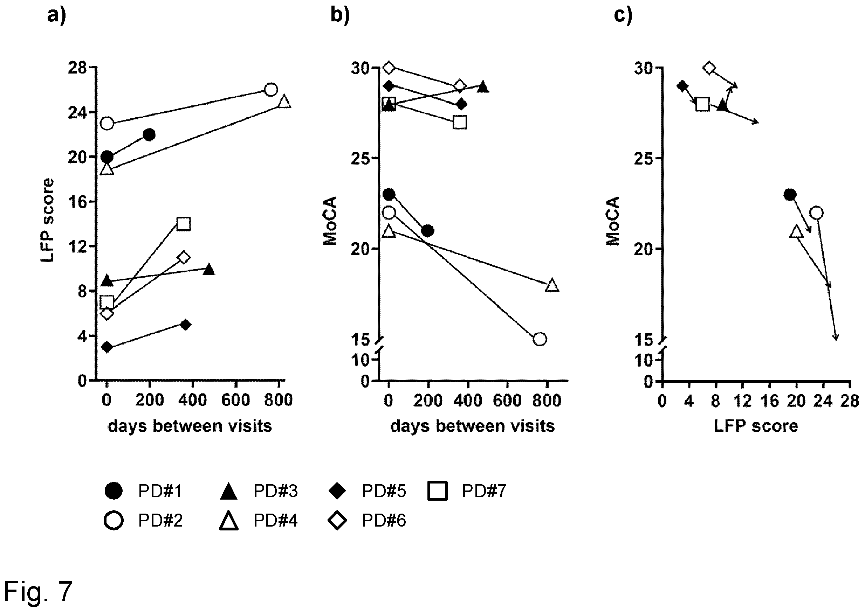

In Fig. 7, a schematic illustration of the temporal connection between the LFP score and MoCA score in PD/DLB cases by longitudinal investigation is depicted. Respective scores from the same patient are connected by a line (left data point = baseline, right data point = follow-up). The x-axis shows the time (days) between visits of baseline and follow-up. Fig. 7a shows an increase in the number of positive replicates during disease progression. In Fig. 7b the change in MoCA from baseline to follow-up of the same patients as in Fig. 7a are plotted. In Fig. 7c the change of MoCA and positive replicates over time are depicted to better visualize the correlation between an increase in LFP score during disease progression and an increased cognitive impairment as presented by a reduction in MoCA score. The start of the arrow is defined by baseline MoCA and LFP score values, whereas the arrow tip ends at the respective values at follow-up, highlighting the association of LFP score and MoCA.

Thus, the present invention is capable of tracking the brain pathology of disease progression, namely an increase in misfolded αSyn, whereas the clinical presentation does normally not change during this timeframe.

In Fig. 8, a schematic illustration of an exemplary αSyn-SAA reaction using brain homogenate as biological sample is depicted. As samples, brain homogenates (phosphate buffered saline (PBS) soluble fraction, 10% weight per volume, centrifuged for 30 min at 10 000 x *g*) from Lewy body disease (LBD; black line, *n* = 10), MSA (+ line, *n* = 10), PSP (dotted line, *n* = 4), and control (healthy individuals, dashed line, *n* = 6) from frontobasal cortex and white matter (Fig. 8a), and cerebellum (Fig. 8b) were used. 2 µl of a 10⁻⁴ dilution of the respective brain homogenates were used alongside 98 µl reaction buffer. RFU values were normalized to the maximum intensity of fluorescence of the respective sample. Each curve represents the average of the group. Standard error of the mean is indicated by dashed pattern. As is known in the art, PD and DLB are taken together neuropathologically as LBD, as both diseases are not distinguishable histologically.

As can be seen in Fig. 8a, no seeding activity above the healthy control group which was used as background could be detected in the frontobasal samples of MSA and PSP. The same is true for cerebellum samples (Fig. 8b). Thus, with the method of the present invention Lewy fold-specific synucleinopathies can be determined and distinguished from MSA synucleinopathies. In addition, the method may be used with diluted and precleared brain homogenate as a sample.

Neuropathologically, the occurrence of pathological αSyn in the frontobasal cortex as well as the cerebellum is much more pronounced in MSA compared to PD. Nevertheless, even in the cerebellum, where MSA shows in contrast to PD/DLB a very strong αSyn pathology, no seeding activity above background in MSA samples as well as in the tested non-synucleinopathy PSP cases could be detected using the inventive method. This shows the high sensitivity and specificity for Lewy fold-specific synucleinopathies.

With the present invention, an improved αSyn SAA with high sensitivity and specificity was developed. A serial dilution of patient samples according to the invention allowed for the differentiation of patients with high vs. patients with low LFP scores. The LFP score correlated with clinical parameters (MoCA, UPDRS III, and Hoehn and Yahr) and mirrored the increase of αSyn pathology during disease progression.

It should be noted that the term "comprising" does not exclude other elements or steps and the "a" or "an" does not exclude a plurality. Further, elements described in association with different embodiments may be combined.

It should also be noted that reference signs in the claims shall not be construed as limiting the scope of the claims.

### Reference numerals

- αSyn: alpha-synuclein
- λ: wavelength
- CSF: cerebrospinal fluid
- SAA: seed amplification assay or seeding aggregation assay
- PD: Parkinson's disease
- DLB: Lewy body dementia
- LBD: Lewy body disease
- MSA: multiple system atrophy
- PSP: progressive supranuclear palsy
- HC: healthy control
- n: number of samples
- AD: Alzheimer's disease
- FTD: Frontotemporal dementia
- LFP: Lewy fold pathology
- neat: undiluted
- p: probability value for significance assessment
- r: Pearson's correlation coefficient
- MDS-UDPRS III: Movement Disorders Society - Unified Parkinson's Disease Rating Scale
- MoCA: Montreal Cognitive Assessment
- RFU: relative fluorescence unit

## Claims

1. A method for detecting misfolded alpha-synuclein protein in a biological sample to determine the presence of synucleinopathies in an individual, wherein the method comprises the steps of:
- S10 contacting a biological sample of the individual with a reaction buffer which comprises soluble monomeric αSyn protein and a fluorophore, to form a reaction mixture;
- S20 incubating the reaction mixture with intermittent shaking/resting cycles;
- S30 subjecting the reaction mixture to excitation;
- S40 determining the fluorescence intensity of the reaction mixture.

2. The method according to claim 1, wherein the biological sample is a body fluid sample or a body tissue sample,
preferably wherein the body fluid sample is selected from cerebrospinal fluid, CSF, blood, serum, plasma, saliva, urine, feces, lymph, preferably CSF,
and/or wherein the body tissue sample is selected from mucosa, nasal swap, skin biopsy, post mortem brain homogenate.

3. The method according to any one of the preceding claims, wherein the method comprises using replicates, preferably doublets, triplets or quadruplets of the biological sample.

4. The method according to any one of the preceding claims, wherein the fluorescence intensity is determined every 45 - 75 minutes, preferably every 55 - 65 minutes, more preferably every 60 minutes.

5. The method according to any one of the preceding claims, wherein the reaction mixture further comprises beads,
preferably wherein the beads comprise zirconia, silica, glass, quartz, and/or combinations thereof, preferably glass beads and/or
wherein the beads have a mean diameter in the range of 0.01 mm to 1 mm, preferably of 0.1 mm to 0.9 mm, more preferably of 0.2 mm to 0.85 mm, and most preferably about 0.8 mm and/or
wherein the reaction mixture comprises 1 to 8 beads per 100 µl, preferably 2 to 7 beads per 100 µl, and more preferably 6 beads per 100 µl.

6. The method according to any one of the preceding claims, wherein the reaction buffer comprises a pH in the range of pH 6.6 - pH 9, preferably pH 7.0 - pH 8.5, more preferably pH 8.0.

7. The method according to any one of the preceding claims, wherein the monomeric αSyn in the reaction buffer is a recombinant wildtype, WT, human αSyn, a variant thereof, or a fragment thereof.

8. The method according to any one of the preceding claims, wherein the monomeric αSyn comprises a polyHis tag on its C-terminus or its N-terminus, preferably the monomeric αSyn comprises an N-terminal polyHis tag.

9. The method according to any one of the preceding claims, wherein the concentration of the monomeric αSyn in the reaction buffer is in the range of 0.01 mg/ml - 0.095 mg/ml, preferably 0.03 mg/ml - 0.09 mg/ml, more preferably 0.04 mg/ml - 0.08 mg/ml, and most preferably 0.07 mg/ml.

10. The method according to any one of the preceding claims, wherein the fluorophore comprises one or more of: thioflavin T, ThT, thioflavin S, ThS, Congo Red, Thiazole Orange, SYBR green, Crystal Violet, 1-anilino-8-naphthalene sulfonate, ANS, and the like, preferably ThT,
and/or wherein the concentration of the fluorophore in the reaction buffer is in the range of 2 µM to 25 µM, preferably 2.5 µM to 20 µM, more preferably 5µM to 15 µM, still more preferably 7 to 11 µM and most preferably 8 µM.

11. The method according to any one of the preceding claims, wherein an increase in the fluorescence intensity indicates the presence of misfolded αSyn in the biological sample.

12. The method according to claim 11, wherein a replicate is considered positive if the Fₘₐₓ reaches 5 - 30% of the average Fₘₐₓ of a positive control, preferably 10 - 25% more preferably 15 - 20%, even more preferably 15%, and most preferably 20%.

13. The method according to any one of the preceding claims, wherein the reaction buffer comprises NaCl in the range of 10 mM - 500 mM, preferably 20 mM - 400 mM, more preferably 50 mM - 200 mM, and most preferably 150 mM,
and/or wherein the reaction buffer comprises sodium phosphate, NaPᵢ, in a concentration in the range of 10 mM to 250 mM, preferably 35 mM,
and/or wherein the reaction buffer comprises sodium dodecyl sulfate, SDS, in a concentration of 0.0008% to 0.0030%, preferably 0.0010% to 0.0026%, more preferably 0.0013%.

14. The method according to any one of the preceding claims, wherein the synucleinopathies are Lewy fold specific synucleinopathies, preferably wherein the Lewy fold specific synucleinopathies are selected from Parkinson's disease, PD, Parkinson's Disease Dementia, PDD, and Dementia with Lewy Bodies, DLB.

15. The method according to any one of the preceding claims, wherein before step S10, the method further comprises:
- S5 diluting the biological sample in a negative control sample, preferably
wherein the dilution comprises a serial dilution comprising undiluted biological sample and/or dilutions of 1:2 and/or 1:3 and/or 1:4 and/or 1:8 and/or 1:10 and/or 1:30 and/or 1:100.

16. The method according to claim 15, wherein the method comprises after step S40:
- S50 counting the number of positive signals obtained from the serial dilution of the biological sample, and scoring these numbers to obtain a Lewy fold pathology, LFP, score; and
- S60 correlating the scores to clinical parameters, preferably
wherein the clinical parameters comprise UPDRS III, Unified Parkinson's Disease Rating Scale, MoCA, Montreal Cognitive Assessment and/or Hoehn and Yahr, symptom progression in PD.

17. A kit for detecting misfolded αSyn protein in a biological sample, wherein the kit comprises a reaction mixture comprising monomeric αSyn, a variant thereof, or a fragment thereof.

18. The kit according to claim 17, further comprising sodium phosphate, an SDS solution, a NaCl solution and water, filter, beads, a fluorophore, a positive control, a negative control, one or more well plate(s) and an adhesive foil for covering of the one or more plate(s).
